Europäisches Patentamt

European Patent Office

Office européen des brevets

(11) Publication number: **0 231 641**
A1

# EUROPEAN PATENT APPLICATION

(21) Application number: 86309980.0

(22) Date of filing: 19.12.86

(51) Int. Cl.³: **A 61 K 39/42**
C 12 P 21/00, C 07 K 15/00
C 12 N 5/00
//C12N15/00, (C12P21/00,
C12R1:91)

(30) Priority: 09.01.86 US 817429

(43) Date of publication of application:
12.08.87 Bulletin 87/33

(84) Designated Contracting States:
AT ES GR

(71) Applicant: THE UPJOHN COMPANY
301 Henrietta Street
Kalamazoo, Michigan 49001(US)

(72) Inventor: Marchioli, Carmine C. The Upjohn Company
301 Henrietta Street
Kalamazoo Michigan 49001(US)

(72) Inventor: Yancey, Robert J. The Upjohn Company
301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

(54) Pseudorabies virus vaccine.

(57) Provided are monoclonal antibodies to pseudorabies virus (PRV) glycoproteins useful for passive immunization of swine against PRV infection.

EP 0 231 641 A1

## PSEUDORABIES VIRUS VACCINE

FIELD OF INVENTION

This invention relates to a passive vaccine for pseudorabies virus. More specifically, the invention relates to monoclonal antibodies to pseudorabies virus glycoproteins, useful in alleviating pseudorabies virus infections in swine.

BACKGROUND OF THE INVENTION

Pseudorabies virus (PRV) is a disease which infects many species of animals worldwide. PRV infections are variously called infectious Bulbar paralysis, Aujeszky's disease, and mad itch. Infections are known in important domestic animals such as swine, cattle, dogs, cats, sheep, rats and mink. The host range is very broad and includes most mammals and, experimentally at least, many kinds of birds (for a detailed list of hosts, see D.P. Gustafson, "Pseudorabies", in Diseases of Swine, 5th ed., A.D. Leman et al., eds., (1981)). For most infected animals the disease is fatal. Adult swine and possibly rats, however, are not killed by the disease and are therefore carriers.

Populations of swine are particularly susceptible to PRV. Although the adult swine rarely show symptoms or die from the disease, piglets become acutely ill when infected and death usually ensues in 24 to 48 hours often without specific clinical signs (T.C. Jones and R.D. Hunt, Veterinary Pathology, 5th ed., Lea & Febiger (1983)).

PRV vaccines have been produced by a variety of techniques and vaccination in endemic areas of Europe has been practiced for more than 15 years. Losses have been reduced by vaccination, but vaccination has maintained the virus in the environment. No vaccine has been produced that will prevent infection. Vaccinated animals that are exposed to virulent virus survive the infection and then shed more virulent virus. Vaccinated animals may therefore harbor a latent infection that can flare up again. (See, D.P. Gustafson, supra).

Live attenuated and inactivated vaccines for PRV are available commercially in the United States and have been approved by the USDA (See, C.E. Aronson, ed., Veterinary Pharmaceuticals & Biologicals, (1983)).

PRV is a herpesvirus. The herpesviruses generally are among the most complex of animal viruses. Their genomes encode at least 50 virus specific proteins and contain upwards of 150,000 nucleotides. Among the most immunologically reactive proteins of herpesviruses are the glycoproteins found, among other places, in virion membranes and the membranes of infected cells. The literature on PRV glycoproteins refers to at least four viral glycoproteins (T. Ben-Porat and A.S. Kaplan, Virology, 41, pp. 265-73 (1970); A.S. Kaplan and T. Ben-Porat, Proc. Natl. Acad. Sci. USA, 66, pp. 799-806 (1970)). Based on research on herpes simplex virus type 1 (HSV-1) (B. Norrild, Curr. Topics Microbiol. Immunol., 90, pp. 67-106 (1980)), the glycoproteins present on the surface of PRV infected cells and virions are believed to be the targets of humoral and cell-mediated immune responses in the infected host.

INFORMATION DISCLOSURE

The study of PRV glycoproteins with monoclonal antibodies has recently been described by M.W. Wathen and L.K. Wathen, J. Virol., 51, pp. 57-62 (1984) who refer to a PRV containing a mutation in a viral glycoprotein (gp50), a method for selecting the mutant utilizing neutralizing monoclonal antibody directed against gp50, and a monoclonal antibody directed against gp50. Wathen and Wathen also indicate that their monoclonal antibody directed against gp50 is a strong neutralizer of PRV, with or without the aid of complement, and that polyvalent immune serum is highly reactive against gp50, therefore concluding that gp50 may be one of the important PRV immunogens. On the other hand, it has been reported that monoclonal antibodies that react with the 98,000 MW envelope glycoprotein (gIII) neutralize PRV infectivity but that monoclonal antibodies directed against some of the other membrane glycoproteins (e.g., gII) have very little neutralizing activity (H. Hampl, et al., J. Virol., 52, pp. 583-90 (1984); and T. Ben-Porat and A.S. Kaplan, "Molecular Biology of Pseudorabies Virus", in B. Roizman ed., The Herpesviruses, 3, pp. 105-73 (1984)).

T.C. Mettenleiter et al., J. Virol., 53, pp. 52-57 (1985), also refer to the use of monoclonal antibodies to map a gene encoding a PRV glycoprotein called gI. Monoclonal antibodies have also been used to partially characterize several PRV glycoproteins with respect to glycosylation and synthesis within infected cells (Hampl et al.,

supra.; and N. Lukacs et al., J. Virol., 53, pp. 166-73 (1985)(gI and gII)).

N.S. Balachandran, et al., Infect. Immun., 37, pp. 1132-37 (1982) and R.D. Dix, et al., Infect. Immun., 34, pp. 192-99 (1981) refer to protecting mice from homologous virus challenge using passive immunization with monoclonal antibodies reactive with herpes simplex virus glycoproteins. D.P. Gustafson et al., J. Am. Vet. Med. Assoc., 160, pp. 623-28 (1972) refer to passive administration of PRV hyperimmune serum and gamma globulin preparations and their effectiveness as a short-term prophylaxis against Aujesky's disease in swine. R.A. Crandell et al., J. Am. Vet. Med. Assoc., 171, pp. 59-63 (1977) refers to suckling pigs given hyperimmune serum incurring a lower rate of mortality than control pigs after exposure to PRV.

The short-term prophylactic treatment of the instant invention is useful after exposure of a herd of pigs to PRV. Highly susceptible young pigs must be protected at that time in order to survive. Vaccination programs would then be started after initial protection is achieved to protect the pigs for a longer time.

The monoclonal antibodies of the instant invention are more advantageous than hyperimmune serum or gamma globulin in a passive immunization program for such protection because they are easier to mass produce and have greater specificity as well as less chance of passing on incidental infection.

SUMMARY OF THE INVENTION

The present invention relates to a method of treating PRV infection in swine by administering an anti-PRV effective amount of a composition comprising a monoclonal antibody against PRV gII, gIII, or gp50 to a swine in need of such treatment.

More particulary, the invention relates to a method of treating PRV infection in swine by administering an anti-PRV effective amount of a composition comprising a monoclonal antibody selected from the group consisting of 3A4, 3D1, and 3D11 to a swine in need of such treatment.

The present invention also relates to a monoclonal antibody selected from the group consisting of 3D11, 3D1, and 3A4.

The present invention also relates to a hybridoma selected from the group consisting of hybridomas 3A-4C, 3D-1C, and 3D-11C.

The present invention also relates to a vaccine for passive immunization of swine against PRV infection comprising a monoclonal antibody against PRV gII, gIII, or gp50.

More particularly, the invention relates to a vaccine for passive immunization of swine against PRV infection comprising a monoclonal antibody selected from the group consisting of 3A4, 3D1, and 3D11.

DETAILED DESCRIPTION OF THE INVENTION

To make the monoclonal antibodies of the instant invention one can use any of the techniques well known to those skilled in the art, e.g., as set forth in R. Kennett, Monoclonal Antibodies, Plenum Press, (1980). We fused spleen cells from an immune mouse with Sp2/0-Ag14 tumor cells. Hybrid cell growth was visible in 145 of 720 microtiter wells initially seeded after cell fusion. The supernatant from each growth-positive well was assayed for the presence of neutralizing antibody by a microneutralization assay (T.C. Holland et al., J. Virol., 45, pp. 672-82 (1983)). Neutralizing activity was detected in 10 of the initial 145 growth-positive wells. The hybridomas that were secreting neutralizing antibody were subsequently cloned by limiting dilution and their isotypes were determined by the Elisa method, supra.

The footpad model of peripheral inoculation of virus was used in the passive immunization studies in mice because the ascending neurological illness associated with this model resembles the pathogenesis of PRV in swine. Footpad inoculation of mice with PRV results in pruritus within 3 to 7 days, followed by ascending myelitis, encephalitis, and death (H.J. Field and T.J. Hill, J. Gen.-Virol., 23, pp. 145-57 (1974)). Although it has been shown that the course of viral infection in mice by HSV can be abrogated by administration of protective antibody either prior to or soon after viral infection (Dix et al., supra. and Balachandran et al., supra.), this is the first report which describes the use of monoclonal antibodies to passively transfer immunity against PRV in swine (throughout the specification and claims, the terms swine and pig(s) are used synonymously).

EXAMPLE 1

1.   Cell Cultures, Media and Virus

Sp2/0-Ag14 cells (M. Shulman et al., Nature, 276, pp. 269-70 (1978); American Type Culture Collection, ATCC CRL 1581)) were grown in Dulbecco's Modified Eagle Medium (DMEM) supplemented with 10% fetal bovine serum, penicillin (100 units/ml), streptomycin (100 µg/ml) and 8-azaguanine (10 µg/ml). The Sp2/0-Ag14 cells were then maintained in the absence of 8-azaguanine for four to six days prior to fusion with spleen cells.

Selection medium used after fusion of spleen cells and Sp2/0-Ag14 cells comprised DMEM supplemented with 20% Zeta Serum (AMF Biological and Diagnostic Products Co., Seguin, TX), hypoxanthine (13.6 µg/ml), aminopterin (0.18 µg/ml), and thymidine (3.9 µg/ml).

Hybrid cells shown to secrete neutralizing antibody were cloned in medium comprised of DMEM supplemented with 20% fetal bovine serum, hypoxanthine (13.6 µg/ml), and 20% conditioned medium obtained from SP2/0-Ag14 cell cultures (cloning medium).

Vero (African Green Monkey Kidney) cells were grown in Eagle Minimum Essential Medium supplemented with 10% fetal bovine serum, penicillin (100 units/ml), streptomycin (100 µg/ml), and fungizone (0.25 µg/ml).

PRV (Rice strain) was propagated in primary rabbit kidney cells as described by Rea et al., J. Virol., 54, pp. 21-29 (1985).

2. Mouse Immunization

A female Balb/c mouse (Charles River Breeding Laboratories, Inc., Wilmington, MA), 5-6 weeks old, was injected subcutaneously with 0.2 ml of a soluble, inactivated PRV-infected cell extract (PR-VAC; Norden Laboratories, Lincoln, NE) on Day 0. The mouse was given booster injections subcutaneously with the same preparation on Day 14 and challenged with PRV (Rice Strain) intraperitoneally on Day 21 with a dose approximately 20 times the 50% lethal dose. A final intraperitoneal injection of 1 ml of soluble PRV-infected cell extract was given on Day 43, three days prior to fusion (Day 46).

3. Fusion and Cloning

Spleen cells obtained from the immune mouse (above) were fused with Sp2/0-Ag14 cells essentially using the procedure described by R. Kennett, Monoclonal Antibodies, Plenum Press, (1980). Spleen cells and Sp2/0-Ag14 cells were mixed at a ratio of 2:1 and washed twice with DMEM. After the final centrifugation, the cells were dispersed and the centrifuge tube containing the cell mixture was placed in a heating block at 40°C. One ml of 40% (w/v) polyethylene glycol 1450 (J.T. Baker Chemical Co., Phillipsburg, NJ) was added at a rate of 0.25 ml/15 sec. The cell mixture was then incubated at 40°C for 1 min followed by addition of 1 ml of DMEM at a rate of 0.25 ml/15 sec. An additional 10 ml of DMEM was immediately added at a rate of 0.5 ml/15 sec. During all additions of polyethylene glycol and DMEM the cell mixture was periodically agitated to insure mixing.

The cell mixture was subsequently centrifuged for 7 min at 1000 RPM. The cells were then dispersed and DMEM supplemented with 20% Zeta Serum was added. Cells were transferred by pipette into 96-well tissue culture plates so that each well received the equivalent of 2 x $10^5$ spleen cells and 1 x $10^5$ Sp2/0-Ag14 cells.

The plates were incubated at 37°C in a humidified incubator with 5% $CO_2$. One drop of selection medium was placed in each well on each of three consecutive days after fusion. Subsequently, the wells were fed weekly by replacing 75% of the medium with fresh selection medium.

Supernatants from growth-positive wells were assayed by a microneutralization assay which is described below. Hybrid cell cultures secreting neutralizing antibody were subsequently transferred to cloning medium and cloned by limiting dilution. Ascites fluids were produced by intraperitoneal injection of 5 x $10^6$ hybrid cells into each of several pristane-primed Balb/c mice.

4. Microneutralization Assay

A microneutralization assay, as described by T.C. Holland et al., _J. Virol._, 45, pp. 672-82 (1983), was used to screen hybrid cell supernatants for neutralizing activity and to determine the neutralization titers of supernatants and ascites fluids. 50 μl of each supernatant or serially diluted ascites fluid was added to 50 μl of virus (100 times the 50% tissue culture infectious dose) and 20% rabbit complement. The microtiter plates were incubated for 3 hrs at 37°C and 3 x $10^4$ Vero cells per well were added. The microtiter plates were then incubated for 48 hrs at 37°C.

Cells were stained with 0.025% crystal violet and the antibody titer was expressed as the reciprocal of the highest dilution of supernatant or ascites which protects greater than 50% of the cells from cytopathic effects. Supernatants obtained from initial growth-positive hybrid cultures were determined to have neutralizing activity if greater than 50% of the cells were protected from cytopathic effects.

5. Immunoprecipitation

Immunoprecipitation of radiolabeled viral proteins with monoclonal antibodies was according to the method of T.J. Rea, et al. J.Virol., 54, 21-29 (1985).

6. Isotype Determination

The isotype of monoclonal antibody secreted by each hybrid cell line was determined by an enzyme-linked immunosorbent assay using a commercially available isotyping kit (Hyclone Laboratories, Logan, UT). This assay was conducted according to the manufacturer's specifications. Of the four finally selected hybridomas, two secreted $IgG_{2a}$, one secreted $IgG_{2b}$, and one secreted IgA (Table 1). Monoclonal antibodies 3A4, 3D1, 3D11, and 2A2 neutralized PRV only in the presence of complement.

Immunoprecipitations using either supernatants or ascites fluids of hybrid cell lines demonstrated that each monoclonal antibody was specific for gII, gIII (gp82), or gp50 (the nomenclature is that of Hampl et al., supra, and Wathen and Wathen, supra.). Immunoprecipitation patterns of monoclonal antibodies 3D11, 3A4, 3D1, and 2A2 were also determined by immunoprecipitation of $^{14}$C-glucosamine-labeled--infected-cell polypeptides using the monoclonal antibodies. The specificities are set forth in Table 1.

Table 1

Monoclonal Antibodies

| Monoclonal Antibody | Isotype | Neutralization[a] | | Specificity[b] | |
|---|---|---|---|---|---|
| | | +C | -C | Wathen | Hampl |
| 3A4 | $IgG_{2a}$ | + | - | gp50 | |
| 3D1 | $IgG_{2b}$ | + | - | | gII |
| 3D11 | $IgG_{2a}$ | + | - | gp82 | gIII |
| 2A2 | IgA | + | + | gp50 | |

[a] Supernatants obtained from hybrid cultures were determined to have neutralizing activity (in the presence or absence of complement) if greater than 50% of the cells were protected from cytopathic effects.
[b] Specificity was determined by immunoprecipitation.

Hybridomas 3A-4C (UC HB-7), 3D-1C (UC HB-13), and 3D-11C (UC HB-14) which produce monoclonal antibodies 3A4, 3D1, and 3D11 respectively and exemplify the invention, were deposited with the American Type Culture Collection, 12301 Parklawn Drive, Rockville, Maryland 20852 on December 18, 1985, and have been assigned accession numbers HB8980, HB8981, and HB 8982 respectively (Note: UC is a registered trademark of The Upjohn Co.).

EXAMPLE 2

In this example we demonstrate that administration of the monoclonal antibodies of Example 1 protected mice from footpad challenge with PRV.

1. Monoclonal Antibodies

The production of hybridomas and the generation of ascites fluids is described in Example 1.

2. Microneutralization Assay

The microneutralization assay used to determine the neutralization titers of ascites fluids is described in Example 1.

3. Passive Transfer Studies

The administration of ascites fluids and subsequent challenge of mice with PRV was conducted as described by N.S. Balachandran et al., supra, with several modifications. Female CF-1 mice, 6-7 weeks old, were each administered 1 ml of the respective antibody or control preparations by the intraperitoneal route. Three hours after administration of the respective preparations mice were challenged by the footpad route with a volume of 0.05 ml containing PRV at 20 times the 50% lethal dose ($LD_{50}$). Mice were observed for mortality for a period of 21 days.

The results of these passive transfer studies are shown in Table 2. The most protective monoclonal antibodies were those specific for PRV glycoproteins gp50 or gIII. Monoclonal antibody 3A4, reactive with gp50, was especially protective in both studies 1 and 2 (90% and 100% survival, respectively). Mice that were administered saline or ascites fluids with no antibody (saline, Sp2/0, and hybridoma control groups) were not protected from PRV infection. In addition, monoclonal antibody 3D1, which is specific for PRV glycoprotein gII, was not protective despite those antibodies having a significant neutralization titer (Tables 2 and 3).

In Studies 1 and 2 monoclonal antibody 3A4 was the most protective, even though monoclonal antibody 3D11 had higher neutralization titers. Monoclonal antibody 3D11 neutralized in the absence of complement (neutralization titer = 40) at a very low level. Neutralization by the other monoclonal antibodies was dependent on the presence of complement.

A challenge dose of 20 $LD_{50}$ was used in each of the two passive transfer studies. The mean day of death (MDD) observed for control mice that were administered Sp2/0 and hybridoma control ascites fluids in Study 1 were each 4.0 days, whereas in Study 2 the MDD for mice that were administered hybridoma control ascites fluid and saline were 5.8 and 7.1 days, respectively. Since the $LD_{50}$ determination is only an approximation, the actual challenge doses in Studies 1 and 2 were probably different, therefore accounting for the slight variation in MDD for control mice between studies. This explanation may also account for the differences in the survival of mice given monoclonal antibodies 3A4 and 3D11 when comparing Studies 1 and 2. These passive transfer studies indicated that monoclonal antibodies directed against gIII and gp50 are protective, whereas the monoclonal antibodies directed against gII do not protect mice against PRV challenge.

Table 2

Mortality of Mice[a]

| Preparation | Dead/ Total | MDD[b] |
|---|---|---|
| Study 1 | | |
| 3A4 | 1/10 | 5.0 |
| 3D1 | 10/10 | 4.0 |
| 3D11 | 5/10 | 7.4 |
| Conv. Pig Serum[c] | 0/10 | --- |
| Sp 2/0 Control[d] | 10/10 | 4.0 |
| Hybridoma Control[e] | 10/10 | 4.0 |
| Study 2 | | |
| 3A4 | 0/10 | --- |
| 3D11 | 1/10 | 8.0 |
| 2A2 | 4/10 | 8.3 |
| Conv. Pig Serum | 1/10 | 10.0 |
| Hybridoma Control | 9/10 | 5.8 |
| Saline Control | 9/10 | 7.1 |

[a]Mice were challenged by the footpad route with PRV at a dose of 20 LD50 3 hrs after administration of the antibody or control preparations.

[b]MDD: mean day of death.

[c]Convalescent pig serum: serum from a recovered PRV-infected pig.

[d]Sp 2/0 control: ascites fluid from mice injected with Sp 2/0 cells.

[e]Hybridoma control: ascites fluid from mice injected with a hybridoma cell line not secreting antibody.

Table 3

Properties of Preparations Passively Administered to Mice

| Preparation | Isotype | Specificity | Neutralization Titer[a] +C | -C | % Survival |
|---|---|---|---|---|---|
| Study 1 | | | | | |
| 3A4 | $IgG2_a$ | gp50 | 327,680 | <20 | 90 |
| 3D1 | $IgG2_b$ | gII | 10,240 | <20 | 0 |
| 3D11 | $IgG2_a$ | gIII | 655,360 | 40 | 50 |
| Conv. Pig Serum | --- | --- | 20,480 | 640 | 100 |
| Sp 2/0 Control | --- | --- | <20 | <20 | 0 |
| Hybrid Control | --- | --- | <20 | <20 | 0 |
| | | | | | |
| Study 2 | | | | | |
| 3A4 | $IgG2_a$ | gp50 | 327,680 | <20 | 100 |
| 3D11 | $IgG2_a$ | gIII | 655,360 | 40 | 90 |
| 2A2 | IgA | gp50 | 2,560 | 2,560 | 60 |
| Conv. Pig Serum | --- | --- | 20,480 | 640 | 90 |
| Hybrid Control | --- | --- | <20 | <20 | 10 |
| Saline Control | --- | --- | <20 | <20 | 10 |

[a]The neutralization titer (+ or - complement) expressed as the reciprocal of the highest dilution of each preparation that protected greater than 50% of the cells from cytopathic effects.

## EXAMPLE 3

In this example we demonstrate that passive immunization of swine with the monoclonal antibodies of Example 1 protects swine from PRV infection.

1. Animals

Study 1: Thirty-six crossbred pigs, 5-6 weeks old and of mixed sex, randomly allotted into six groups (six pigs/group), were used. Sixteen pigs of the same age were also used for the determination of the 50% lethal dose. Swine were given unmedicated feed and water ad libitum.

Study 2: Thirty crossbred pigs, 5-6 weeks old and of mixed sex, randomly allotted into five groups, were used. Sixteen pigs of the same age were also used to determine the LD50.

2. Production of Antibody Preparations

Study 1: Ascites fluids were prepared by injecting $5 \times 10^6$ cells of each hybridoma cell line into separate pristane-primed mice. After the appearance of an ascites tumor the mice were tapped for ascites fluids on alternate days until death.

Study 2: The monoclonal antibodies were prepared by culturing hybridoma cell lines in 16-liter-suspension culture vessels operated as 10 liters. Each 10-liter harvest was concentrated to 1 liter with a Pellicon Ultrafiltration unit using 10,000 MW cut-off membranes. The concentrate was brought to 4°C and 1 liter of saturated ammonium sulfate at 4°C was added slowly under vigorous stirring, allowed to stand for 1 hour, and centrifuged at 3000 xg. The precipitate was placed in Spectra/Por 6 dialysis tubing (2.7 mm dia.) and dialyzed for 24 hours at a 1:100 dialysate:buffer volume ratio. The buffer was replaced once, giving a total of 48 hours for dialysis and a $10^4$ reduction of salts. Dialysis buffer was 0.15 M NaCl in Milli-Q quality water. Each dialysate was then sterile filtered through 0.45 $\mu$ filters and later divided into 6 doses in 50 ml sterile serum bottles.

3. Determination of 50% Lethal Dose (LD50)

Sixteen pigs randomly alloted into four groups (four pigs/-group), were used to determine the LD50 of the Rice strain of PRV. The four groups received $10^4$, $10^3$, $10^2$, and $10^1$ plaque forming units (pfu)/pig, respectively, by intranasal inoculation. The LD50 was determined by the method of L. J. Reed and H. Muench, Am. J. Hygiene, 27, pp. 493-97 (1938).

4. Virus Isolation from Nasal Swabs

Nasal swabs collected from pigs were each placed in one ml of Eagles Basal Medium (BME; M.A. Bioproducts) supplemented with 3% fetal bovine serum (FBS) and antibiotics. Swabs were stored at -70° until they were assayed for the presence of virus. For the virus isolation assay, the nasal swabs in BME were thawed and the individual swabs were discarded. Samples (0.1 ml) were inoculated in duplicate onto porcine kidney-15 (PK-15; ATCC CCL33) cell monolayers and incubated for 1 hr at 37° to allow virus adsorption. An overlay of medium-199 (Flow Laboratories) supplemented with 4% FBS, antibiotics, and 1% agar was placed on the cell cultures. After 3 days the cell monolayers were stained with neutral red and the plaques were enumerated.

5. Passive Transfer

Study 1: Pigs were treated as outlined in Table 5. Pigs in groups 1 through 3 were administered 5 ml ascites fluid containing monoclonal antibodies (3A4, 3D11, or 3D1) by the intraperitoneal (IP) route. The amount of immunoglobulin was not quantitated in this study. Pigs in group 4 served as positive controls and received 5 ml serum obtained from a pig recovered from PRV infection (convalescent pig serum). Pigs in group 5 served as negative controls and received 5 ml ascites fluid containing no monoclonal antibody.

All pigs were challenged intranasally with 20 times the 50% lethal dose (20 $LD_{50}$ = 3.8 x $10^4$ plaque forming units/pig) of PRV (Rice strain) 4 hrs after administration of the antibody or control preparations on Day 0. Nasal swabs were obtained from each pig daily beginning on Day 2. The study was terminated 21 days after challenge (Day 21).

Study 2: Pigs were administered about 150 mg of the respective antibody preparations by the intraperitoneal route (Table 4). Control pigs were given either fetal calf serum proteins prepared in a manner similar to the monoclonal antibody preparations or convalescent pig serum. Four hours after administration of the respective preparations swine were challenged intranasally with a volume of four ml (two ml/nostril) containing PRV (Rice strain) at 20 times the 50% lethal dose (20 $LD_{50}$ = 5.0x$10^4$ plaque forming units/pig). Swine were observed for mortality for a period of 14 days and nasal swabs were obtained daily form each pig. In addition, swine were weighed prior to challenge (day 0) and at termination (day 14) or death.

Table 4

Composition of Preparations Used in Study 2

| Monoclonal Ab | 3A4 | 3D1 | 3D11 | Fetal Bovine Serum |
|---|---|---|---|---|
| No. of Doses | 6 | 6 | 6 | 6 |
| Volume (ml)/Dose | 25.6 | 26.2 | 33.0 | 32.0 |
| mg/ml IgG | $5.86 \pm 0.54$[a] | $5.73 \pm 0.48$[a] | $4.55 \pm 0.68$[a] | 0 |
| mg/ml Total Protein | $33.8 \pm 0.2$ | $42.8 \pm 1.5$ | $34.3 \pm 1.3$ | 35.3 |
| mg IgG/Dose | $150 \pm 14$[b] | $150 \pm 13$[b] | $150 \pm 23$[b] | 0 |
| mg Total Protein/Dose | $865 \pm 2$ | $1120 \pm 20$ | $1130 \pm 20$ | 1130 |
| (IgG/Total Protein)x100 | $17.3 \pm 1.6$ | $13.4 \pm 1.2$ | $13.2 \pm 2.0$ | 0 |
| Total IgG Produced (mg) | $936 \pm 69$ | $1000 \pm 80$ | $1260 \pm 100$ | -- |
| Percent Recovered | $103 \pm 12$ | $134 \pm 18$ | $78 \pm 13$ | -- |

Total protein determinations were conducted using the Bradford assay (M. Bradford, Anal. Biochem., 72, p. 248 (1976)), with bovine serum albumin as the standard.

[a]Relative standard deviation is a combination of relative standard deviations from two or more independent fractions that were mixed to produce the formulated dose.

[b]Note that the calculated concentrations of IgG have large standard deviations. The IgG concentrations were determined several times and in most instances were calculated to be within two standard deviations of 150 mg.

6. Criteria for Efficacy Evaluation

Efficacy of each monoclonal antibody preparation was evaluated on the basis of protection against PRV-induced mortality.

The results of the study are set forth in Table 5.

Table 5

| Group[a] No. | Preparation[b] | Isotypes | Glycoprotein Specificity | Neutralization Titer[c] | Survivors/ Total |
|---|---|---|---|---|---|
| STUDY 1 | | | | | |
| 1 | mAb 3A4 | IgG$_{2a}$ | gp50 | 40,960 | 2/6 |
| 2 | mAb 3D11 | IgG$_{2a}$ | gIII | 327,680 | 5/6 |
| 3 | mAb 3A4, 3D11, 3D1 | IgG$_{2a}$, IgG$_{2a}$, IgG$_{2b}$ | gp50, gIII, gII | 655,360 | 3/6 |
| 4 | mAb 2A2 | IgA | gp50 | 320 | 0/6 |
| 5 | Convalescent Pig Serum | -- | -- | 163,840 | 4/6 |
| 6 | Ascites Control | -- | -- | <20 | 0/6 |
| STUDY 2 | | | | | |
| 1 | mAb 3A4 | IgG$_{2a}$ | gp50 | 81,920 | 3/5 |
| 2 | mAb 3D11 | IgG$_{2a}$ | gIII | 81,920 | 6/6 |
| 3 | mAb 3D1 | IgG$_{2b}$ | gII | 5,120 | 3/5 |
| 4 | Convalescent Pig Serum | -- | -- | 81,920 | 5/6 |
| 5 | FBS Control[d] | -- | -- | <20 | 1/6 |

[a]Five or six pigs per group.

[b]Pigs were treated as set forth in the text. mAb = monoclonal antibody; FBS = fetal bovine serum.

[c]The neutralization titer is expressed as the reciprocal of the highest dilution of serum that protected greater than 50% of the cells from cpe.

[d]FBS control was used as a negative control for protection and contained no PRV-specific antibody.

Monitoring of virus shedding from nasal secretions demonstrated that virus was shed by pigs administered any monoclonal antibody or control preparation. No difference in viral shedding was apparent between pigs administered specific monoclonal antibody preparations.

Pigs that were given monoclonal antibody preparations did not gain weight as readily as pigs that were given convalescent pig serum. This may have been due to either the effect of convalescent pig serum antibodies in decreasing the onset of morbidity or the adverse effect of contaminants such as ammonium sulfate, phenol red, and bovine serum proteins present in monoclonal antibody preparations.

Monoclonal antibody 2A2 did not protect in this study at the levels tested. While not wishing to bound by theory, this is possibly because it is an IgA immunoglobulin and not IgG as were those that protected. Also, as set forth supra, we did not quantitate the amount of immunoglobulin in Study 1 and therefore only a small amount of 2A2 may have been administered in the experiment.

Monoclonal Antibody as Therapy

1. Composition of vehicle(s) - e.g., water, saline, buffer suspensions of purified and/or processed monoclonal antibody or unaltered ascites fluid.

2. Route of administration - intraperitoneal, intravenous, intramuscular or subcutaneous, oral or intranasal.

3. Dosage regimen - at first signs of pseudorabies in the herd, treat all susceptible (non-vaccinated) pigs. One dose is generally adequate but a second dose may be required in the event of further pseudorabies infections. No more than two doses should be administered due to the possible adverse immune reactions to mouse protein.

4. Duration of protection - for the first dose, up to about 10 days; for the second dose, no more than about one week.

5. Vaccination with commercial vaccines during treatment - administration of vaccines currently available commercially (such as the attenuated virus or inactivated virus vaccines) is contraindicated since the antibody of the monoclonal therapeutic agent will react with the vaccine antigen and cause rapid clearance of antigen and monoclonal antibody. Commercial vaccine may be administered at about seven days post therapy as long as a second dose of monoclonal antibody therapy is not contemplated.

6. Amount of monoclonal antibody - About 20 mg to about 300 mg of monoclonal antibody may be administered per pig. The preferable amount is from about 50 to about 150 mg per pig. Preferably about 1 ml to about 10 ml of a composition comprising the monoclonal antibody will be administered. The maximum administered will be about 50 ml. The amount of material administered will be route dependent and animal dependent (e.g., based on age, size, and general health). Less volume can be administered into the muscle or intravenously than can be administered intraperitoneally or subcutaneously.

We claim:

1. Use of a monoclonal antibody against PRV gII, gIII, or gp50 for the manufacture of a medicament for the treatment of PRV infection in swine.

2. Use according to claim 1 wherein the monoclonal antibody is selected from the group consisting of 3A4, 3D1, and 3D11.

3. A monoclonal antibody selected from the group consisting of 3D11, 3D1, and 3A4.

4. A hybridoma selected from the group consisting of hybridomas 3A-4C, 3D-1C, and 3D-11C.

5. A vaccine for passive immunization of swine against PRV infection comprising a monoclonal antibody against PRV gII, gIII, or gp50.

6. A vaccine according to claim 5 wherein the monoclonal antibody is selected from the group consisting of 3A4, 3D1, and 3D11.

7. A method of treating PRV infection in swine by administering an anti-PRV effective amount of a composition comprising a monoclonal antibody against PRV gII, gIII, or gp50 to a swine in need of such treatment.

8. A method according to claim 1 wherein the monoclonal antibody is selected from the group consisting of 3A4, 3D1, and 3D11.

0231641

Application number

EP  86 30 9980

**European Patent Office**

## PARTIAL EUROPEAN SEARCH REPORT

which under Rule 45 of the European Patent Convention shall be considered, for the purposes of subsequent proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 104, no. 5, February 3, 1986, page 443, ref. no. 32830c; Columbus, Ohio, US L.M.K. WATHEN et al.: "Production and characterization of monoclonal antibodies directed against pseudorabies virus." & VIRUS RES. 1985, 4(1), 19-29. * Abstract * | 1-6 | A 61 K 39/42 C 12 P 21/00 C 07 K 15/00 C 12 N  5/00// C 12 N 15/00 (C 12 P 21/00 C 12 R  1:91) |
| A,D | BIOLOGICAL ABSTRACTS, vol. 78, no. 9, 1984, page 8142, ref.no. 72347; Philadelphia, US M.W. WATHEN et al.: "Isolation, characterization and physical mapping of a pseudorabies virus mutant containing antigenically altered gp50." & J. VIROL., 51(1), 57-62, 1984. * Abstract * | 1-6 | |
| A,D | CHEMICAL ABSTRACTS, vol. 101, no. 23, December 3, 1984, page 330, | | |

**TECHNICAL FIELDS SEARCHED (Int. Cl.4)**

A 61 K

C 12 P

C 12 N

### INCOMPLETE SEARCH ./.

The Search Division considers that the present European patent application does not comply with the provisions of the European Patent Convention to such an extent that it is not possible to carry out a meaningful search into the state of the art on the basis of some of the claims.

Claims searched completely:      1-6
Claims searched incompletely:
Claims not searched:      7,8
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 08-04-1987 | RIJCKEBOSCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 . 03.82

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | ref.no. 207336p; Columbus, Ohio, US H. HAMPL et al.: "Characterization of the envelope proteins of pseudo-rabies virus." & J. VIROL. 1984, 52(2), 583-90. * Abstract * --- | 1-6 | |
| P,X | BIOLOGICAL ABSTRACTS/RRM, vol. 31, 1986, ref.no. 31007477; Philadelphia US C.C. MARCHIOLI et al.: "Protection of mice and swine from pseudorabies virus-induced mortality with mono-clonal antibodies directed against pseudorabies virus glycoproteins." & ABSTRACTS OF THE ANNUAL MEETING OF THE AMERICAN SOCIETY FOR MICRO-BIOLOGY (US), 1986, 86(O), 315. * Abstract * ------------------- | 1-6 | TECHNICAL FIELDS SEARCHED (Int Cl 4) |

EPO Form 1505.3  06.78

European Patent Office

Application 0231641

*96309980.0*

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:  ATCC  HB 8980
HB 8981
HB 8982